Europäisches Patentamt

European Patent Office

Office européen des brevets

(11) Publication number: **0 182 289**
A1

# EUROPEAN PATENT APPLICATION

(21) Application number: 85114478.2

(22) Date of filing: 14.11.85

(51) Int. Cl.⁴: **A 61 B 1/04,** G 02 B 23/24, G 02 B 23/26

(30) Priority: 15.11.84 JP 173896/84 U

(43) Date of publication of application: 28.05.86 Bulletin 86/22

(84) Designated Contracting States: **DE FR GB IT NL SE**

(71) Applicant: SUMITOMO ELECTRIC INDUSTRIES LIMITED, No. 15, Kitahama 5-chome Higashi-ku, Osaka-shi Osaka 541 (JP)

(72) Inventor: Tsuno, Koichi Osaka Works of Sumitomo Electric, Industries, Ltd. No. 1-3, Shimaya 1-chome, Konohana-ku Osaka-shi Osaka (JP)

(74) Representative: Patentanwälte Grünecker, Kinkeldey, Stockmair & Partner, Maximilianstrasse 58, D-8000 München 22 (DE)

(54) **Bidirectional optical adapter.**

(57) A bidirectional optical adapter capable of supplying an image output from a fiberscope (1) includes at least one beam splitter device (5) and a prism (7) for directing luminous flux (1) to separate and distinct image-receiving apparatuses (2, 3). The longer optical path within the adapter includes an optical system (6, 9) for reducing the eclipse effect. Furthermore, the position of the beam splitter (5) or prism (7) (or both) is adjustable in order to overcome system machining inaccuracies.

BIDIRECTIONAL OPTICAL ADAPTER

Field of the Invention

The invention relates to a bidirectional optical adapter capable of simultaneously supplying an image on the image-receiving end face of an image (optical) fiber to two image-receiving apparatuses, such as TV cameras, 16 mm cinecameras and 35 mm cameras.

Background of the Invention

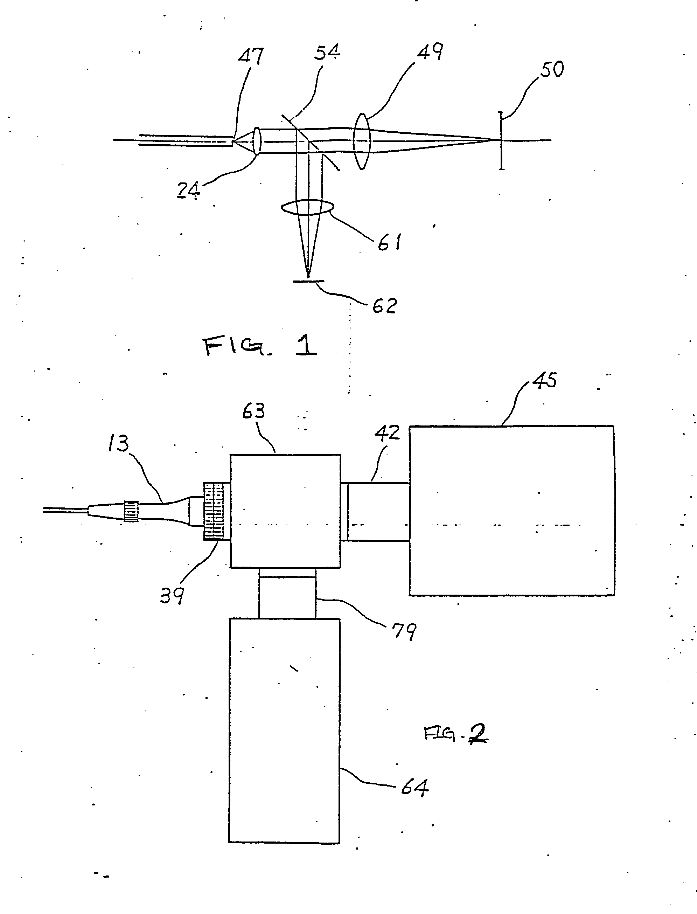

FIGURES 1-6 collectively illustrate the arrangement and operation of prior art bidirectional optical adapters. FIGURE 1 schematically illustrates the configuration of a conventional optical system for actualizing bidirectional optical adaptation as disclosed in Japanese Utility Model No. 59-5040. An image-receiving end face 47 provides luminous flux through lens 24 to half mirror 54. The way that the image is transmitted through and reflected from half mirror 54 to be, respectively, fed onto film 50 and photoelectric surface 62 is illustrative of how the components shown in FIGURE 2 operate. For example, the image reflected from half mirror 54 is formed on photoelectric surface 62 by lens 61 mounted on TV camera 64 and the image transmitted through half mirror 54 is formed on film 50 by lens 49 mounted on 35 mm camera body 45. FIGURE 2 also shows image-receiving adapter 13 connected to the end of a fiberscope and at the same time coupled to 35 mm camera body 45 and TV camera 64, via respective connecting barrels 42 and 79 and through bidirectional optical adapter 63 and image-receiving coupler 39.

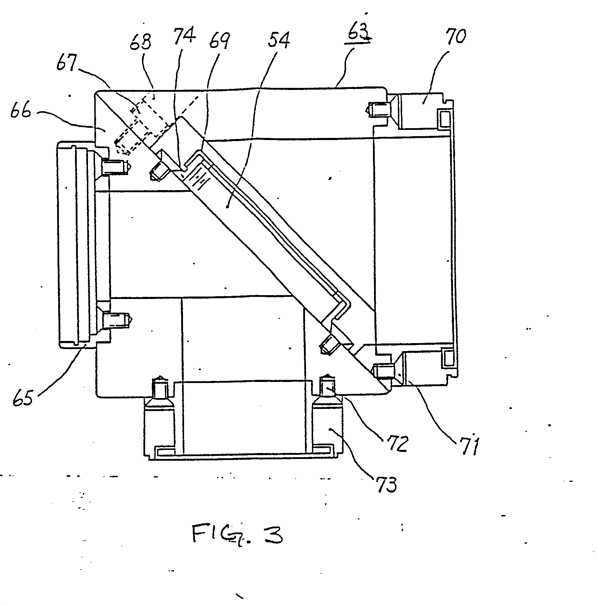

FIGURE 3 is a detailed constructional drawing of bidirectional optical adapter 63. The aforesaid image-receiving coupler 39 is coupled to bidirectional optical adapter 63 with screws from setting adapter 65. The half mirror 54 is fixed to body 66 of bidirectional optical adapter 63 using setting seat 74 and cap 69. In addition, rotary setting adapters 70 and 73 together with screws 71 and 72 are used for fitting optical adapter 63 to the image-receiving apparatuses. There is also shown in broken lines in FIGURE 3 additional setting adapter 68 and screw 67 for assembling optical adapter 63.

With the aforementioned arrangement, image-receiving apparatus 64, receiving the image reflected from half mirror 54, receives an image with the right side flipped to the left and accordingly makes operation, while monitoring the image, particularly inconvenient. Since image-receiving apparatus 45 receives the transmitted image correctly directed, there must be further provided an optical system having another total reflecting mirror 76, as shown in FIGURE 4, to redirect the image to image-receiving apparatus 64. Therefore, a relay adaptor 75 and a reversal optical adaptor 78, shown in FIGURE 5, must be added. FIGURE 6 illustrates a detailed construction of the bidirectional optical adapter. That is, the total reflecting mirror 76 is mounted on the body 77 of the reversal optical adaptor 78.

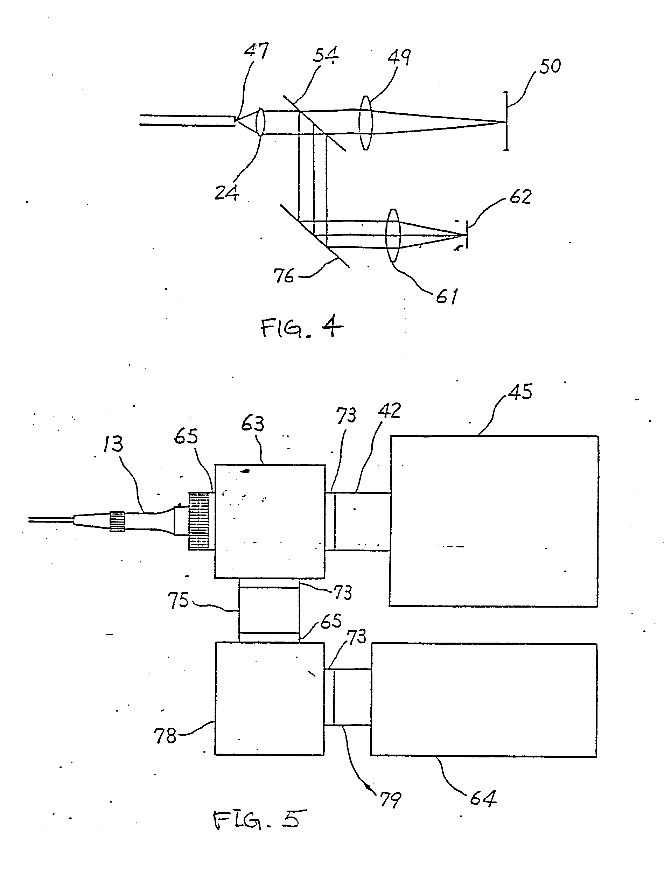

The above-described bidirectional optical adapter has a number of disadvantages. First, in FIGURE 4, the center of the photoelectric surface 62 may divert from the center of the optical axis because of mechanical machining inaccuracy or play produced at the time various parts are coupled together, resulting in the diversion of the image on the image-receiving end face of the image fiber from the center on the TV monitor. Consequently, proper adjustment is impossible with the aforementioned arrangement. Secondly, the light ouput of the image fiber is liable to diverge because the distance from the image-receiving end face 47 up to the photoelectric surface 62 is long and consequently the luminous flux required to form the image from the image-receiving end face 47 to the photoelectric surface 62 is intercepted, that is, " eclipse " of the TV camera lens is produced. For that reason, part of the image on the image-receiving end face may wane on the TV monitor.

## Summary of the Invention

The present invention is intended to solve the above described problems and it is therefore an object of the invention to provide an improved bidirectional optical adapter for an image fiber.

The present invention includes a beam splitting device for receiving a light output from a fiberscope and directing the light output along two separate and distinct optical paths to separate image-receiving apparatuses. An eclipse reduction device is disposed along the longer of the two optical paths as is also a beam redirection device or prism. Either one or both of the beam splitting device and beam redirection device can be mounted for positional adjustment. In this way the assembled invention can be fine tuned (i.e., adjusted) to overcome machining inaccuracies.

## Brief Description of the Invention

FIGURE 1 represents a schematic illustration of a device for accomplishing bidirectional optical adaptation;

FIGURE 2 shows an actual device which operates in accordance with the principles shown in FIGURE 1;

FIGURE 3 shows in detail one component of the FIGURE 2 device;

FIGURE 4 is a modified schematic illustration of the FIGURE 1 device;

FIGURE 5 shows an actual device which operates in accordance with the principles shown in FIGURE 4;

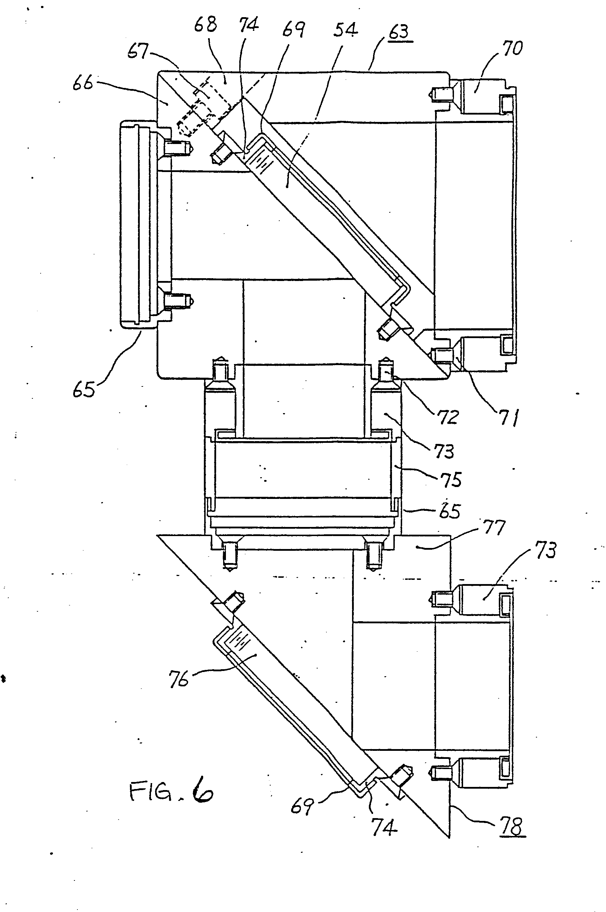

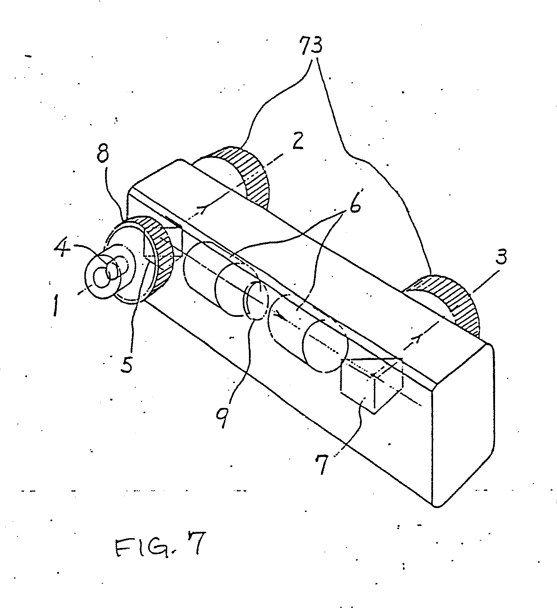

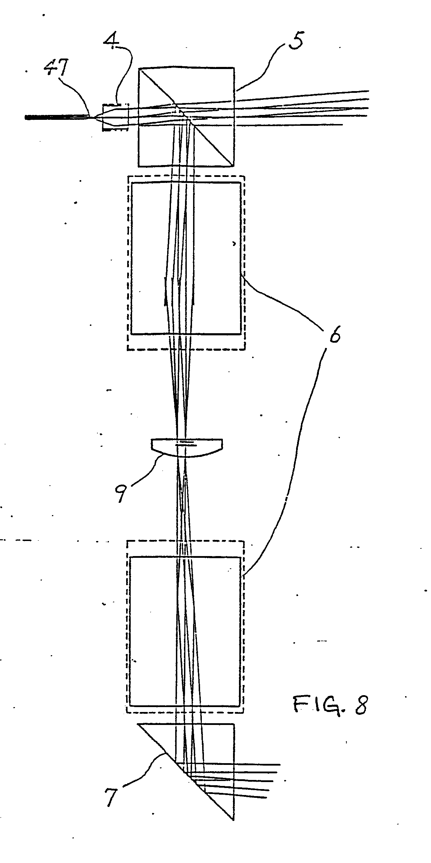

FIGURE 6 shows in detail components of the FIGURE 5 device;

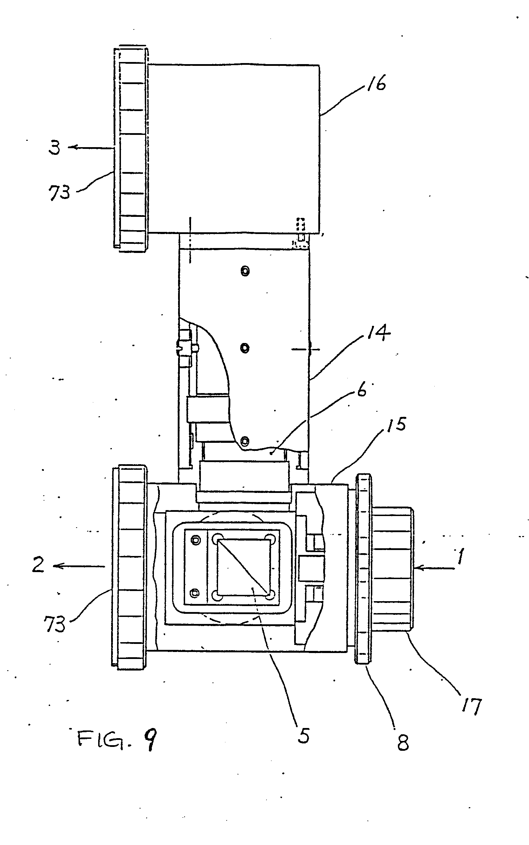

FIGURE 7 shows a perspective view of the present invention;

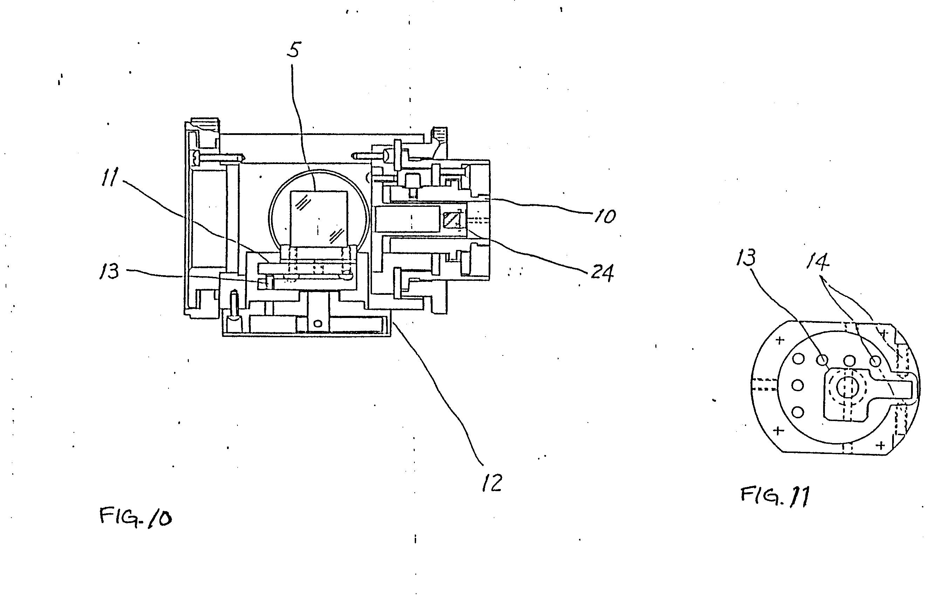

FIGURE 8 shows in detail the arrangement of specific components of the present invention;

FIGURE 9 shows a plane view of the present invention;

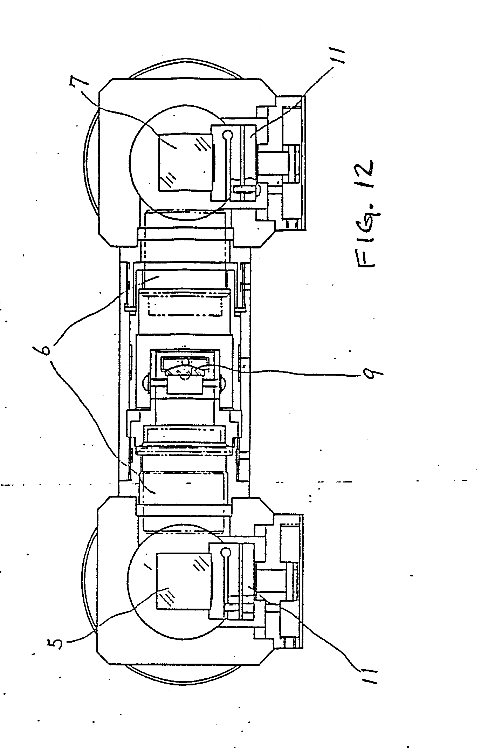

FIGURE 10 shows a front view of a portion of the present invention;

FIGURE 11 shows in detail the swing and tilt adjustment mechanism of the present invention; and

FIGURE 12 shows a side and cut away view of the present invention.

## Detailed Description of the Invention

An embodiment of the present invention will be described with reference to FIGURES 7-12. In FIGURE 7, a light input 1 is made to branch off into first light output 2 and second output 3. The bidirectional optical adapter according to the present invention differs from those in the prior art in that an easily installable cubic beam splitter 5 and 3-sided prism 7 are provided in place of half mirror 54 and total reflecting mirror 66, respectively, in block 15. Moreover, there are provided two intermediate imaging lenses 6 in between beam splitter 5 and prism 7 to reduce " eclipse ". A condenser lens 9 for increasing the effects of the " eclipse " reduction may also be provided close to the imaging position between the two intermediate imaging lenses 6.

FIGURE 8 shows luminous flux entering beam splitter 5 after passing through lens 24. The luminous flux is directed from beam splitter 5 along two separate and distinct optical paths. The luminous flux passes through intermediate imaging lenses 6, condenser 9 and prism 7 along one optical path. As shown in FIGURE 8, the eclipse effect of the luminous flux is reduced when it passes through intermediate imaging lenses 6.

In FIGURE 9, block 16 containing the prism is rotatable on the axis of lens barrel 14 and the left-to-right or other directional alterations of second light output 3 can readily be made. Such a mechanism is quite effective when, for instance, the 35 mm camera and the TV camera are spatially not interchangeable. A focus point adjusting screw 17 and focus point locking screw 8 are used to adjust the focus point. Furthermore, the bidirectional optical adapater is, as shown in FIGURE 9, coupled to the lens of the 35 mm camera and the TV camera through rotary setting adaptor 73, as is the case in the prior art.

FIGURE 10 is a front view illustrating the construction of the optical system covering image-receiving lens 24, within housing 10, up to beam splitter 5. The splitter 5 is set on adjusting table 11 and, by turning a driver (not shown) inserted in a hole made in pedestal 12, a swing screw is made to go up and down so that swing and tilt adjustment on the plane may be provided. Moreover, beam splitter 5 is interlocked with adjusting bar 13 and, by moving two hexagon socket head cap screws from both ousides, the rotation thereof can be adjusted.

As is obvious from FIGURE 11, swing and tilt adjustment in the direction perpendicular to what has been described above can be provided by adjusting table 11 in the same manner as aforementioned. Swing and tilt and rotation adjustment may also be provided for prism 7.

FIGURE 12 is a side view of the bidirectional optical adaptor comprising beam splitter 5, intermediate imaging lenses 6, condenser lens 9 and prism 7.

The above described structural arrangement results in several advantageous effects.  First, with respect to the second light output 3 having a long optical path, decentering is adjustable and thus correctible.  Secondly, the " eclipse " of the luminous flux is reducible.  And finally, since a cubic beam splitter and a 3-sided prism are used, the holding mechanism is made simpler and more compact as compared to the prior art which is equipped with the plate-shaped component tilted at 45°.  Accordingly the present invention is simpler to manufacture and will result in lower production costs.

While a specific  typical  embodiment has been described in detail, numerous changes, modifications and variations may be made without departing from many of the novel advantages and features of the invention.  For example another beam splitter could be added between the first beam splitter and prism in order to obtain three luminous flux outputs. Accordingly, all such changes, modifications and variations are intended to be covered by the following claims.

CLAIMS:

1.  A bidirectional optical adapter capable of supplying
an image output from a fiberscope, containing an image
fiber, to at least first and second image-receiving apparatuses,
said adapter comprising:

an image-receiving lens for receiving said image
output from said fiberscope and for outputting a luminous
flux substantially in parallel with the axial direction of
said fiberscope;

beam splitting means for receiving said luminous
flux output from said image-receiving lens and directing
said luminous flux along at least first and second optical
paths to said at least first and second image-receiving
apparatuses, said beam splitting means being adjustable in
at least one direction for adjusting the position of said
luminous flux along said at least first and second optical
paths;

eclipse   reduction means disposed along one of
said optical paths for reducing the amount of  eclipse   of
said luminous flux directed along said one optical path; and beam redirection means disposed along said one
optical path for receiving said luminous flux from said
 eclipse   reduction means and redirecting it to one of said
at least first and second image-receiving apparatuses, said
beam redirection means being adjustable in at least one
direction for adjusting the position of said luminous flux
along said one optical path.

2.   The adapter as in claim 1, said beam splitting
means comprising a beam splitter.

3.   The adapter as in claim 1, said  eclipse   reduction
means comprising first and second image-receiving lenses.

4.   The adapter as in claim 3, said eclipse   reduction means further comprising a condenser lens disposed between said first and second image-receiving lenses.

5.   The adapter as in claim 1, said beam redirection means comprising a prism.

6.   The adapter as in claim 1, wherein said first optical path leads to a first setting adapter for a lens of said first image-receiving apparatus and said second optical path leads to a second setting adapter for a lens of said second image-receiving apparatus.

7.   The adapter as in claim 6, wherein said first and second setting adapters comprise detachable rings for coupling said first and second setting adapters to said adapter.

8.   A bidirectional optical adapter capable of supplying an image output from a fiberscope, containing an image fiber, to first and second image-receiving apparatuses, said adapter comprising:
       an image-receiving lens for receiving said image output from said fiberscope and for outputting a luminous flux substantially in parallel with the axial direction of said fiberscope;
       a beam splitter for receiving said luminous flux output from said image-receiving lens and directing said luminous flux along first and second optical paths to said first and second image-receiving apparatuses:
       first and second image-receiving lenses disposed along one of said first and second optical paths for reducing the amount of  eclipse   of said luminous flux directed along said one optical path; and

a prism for receiving luminous flux from said first and second image-receiving lenses and redirecting said luminous flux to one of said first and second image-receiving apparatuses.

9.   The adapter as in claim 8, further comprising a condenser lens disposed between said first and second image-receiving lenses.

10.   The adapter as in claim 8, wherein said first optical path leads to a first setting adapter for a lens of said first image-receiving apparatus and said second optical path leads to a second setting adapter for a lens of said second image-receiving apparatus.

11.   The adapter as in claim 10, wherein said first and second setting adapters include detachable rings for coupling said first and second setting adapters to said adapter.

12.   The adapter as in claim 8, further comprising at least one adjustable mounting mechanism for at least one of said beam splitter and prism, said at least one adjustable mounting mechanism adjusting the position of said at least one of said beam splitter and prism in at least one direction.

FIG. 1

FIG. 2

FIG. 3

FIG. 4

FIG. 5

0182289

FIG. 6

FIG. 7

0182289

FIG. 8

769    0182289

FIG. 9

FIG. 11

FIG. 10

FIG. 12

0182289

EUROPEAN SEARCH REPORT

European Patent Office

Application number

EP 85 11 4478

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| Y | EP-A-0 087 033 (OLYMPUS OPTICAL CO., LTD.) <br><br> * Whole document * | 1-3,5, 6,8,10 ,12 | A 61 B 1/04 <br> G 02 B 23/24 <br> G 02 B 23/26 |
| Y | US-A-3 990 778 (R. MAGEE et al.) <br><br> * Column 3, lines 30-35; figure 1 * | 1-3,5, 6,8,10 ,12 | |
| A | OPTICS LASER TECHNOLOGY, vol. 6, no. 2, April 1974, pages 51-52, Haywards Heath, GB; "Rod lens endoscopes" <br> * Figure * | 4,9 | |
| A | US-A-4 413 278 (R. FEINBLOOM) <br> * Abstract * | 7,11 | TECHNICAL FIELDS SEARCHED (Int. Cl.4) |
| A | EP-A-0 076 937 (OLYMPUS OPTICAL CO., LTD.) | | A 61 B 1/00 <br> G 02 B 23/00 <br> G 02 B 27/00 |
| A | US-A-3 995 287 (K. STORZ) | | |

The present search report has been drawn up for all claims

| Place of search | Date of completion of the search | Examiner |
|---|---|---|
| THE HAGUE | 10-02-1986 | POPINEAU G.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82